# EUROPEAN PATENT SPECIFICATION

(11) **EP 4 500 025 B1**
(45) Date of publication and mention of the grant of the patent: **25.02.2026**
(21) Application number: 23715525.4
(22) Date of filing: 30.03.2023
(51) Int. Cl.: F04F 5/16, F04F 5/46, A61B 90/70, B08B 1/00, A61B 90/40, B08B 5/02, A61L 2/20

(54) **ELECTROHYDRODYNAMIC GAS FLOWRATE AMPLIFIER FOR GENERATING A GAS FLOW**
ELEKTROHYDRODYNAMISCHER GASSTRÖMUNGSVERSTÄRKER ZUR ERZEUGUNG EINES GASSTROMS
AMPLIFICATEUR DE DÉBIT DE GAZ ÉLECTROHYDRODYNAMIQUE POUR GÉNÉRER UN FLUX DE GAZ

(30) Priority: 31.03.2022 EP 22165750
(43) Date of publication of application: 05.02.2025
(73) Proprietor: Belimed AG, 6300 Zug (CH); EMPA Eidgenössische Materialprüfungs- und Forschungsanstalt, 8600 Dübendorf (CH)
(72) Inventor: XIE, Lei, 5507 Mellingen (CH); DEFRAEYE, Thijs, 8051 Zürich (CH); RUBINETTI, Donato, 4653 Obergösgen (CH)
(74) Representative: GLP S.R.L.
(86) International application number: PCT/EP2023/058276
(87) International publication number: WO 2023/187046

(56) References cited:
- WO-A1-2011/092755
- US-A1- 2019 242 409
- US-B2- 7 545 640

## Description

The invention relates to a electrohydrodynamic gas flowrate amplifier for generating an gas flow, washer for washing medical instruments comprising a washing chamber and an electrohydrodynamic gas flowrate amplifier, a method if amplifying gas using an electrohydrodynamic gas flowrate amplifier, the use of an electrohydrodynamic gas flowrate amplifier for amplifying gas and the use of an electrohydrodynamic gas flowrate amplifier for disinfection.

Gas flowrate generators for generating gas flows are well known. In the most common way gasflow is generated by ventilators. Gas flowrate generators with ventilators comprise many moving parts and thus, they can break easily and are noisy. Furthermore, moving ventilators create friction and therefore small particles contaminate the accelerated gas. This is a major disadvantage, for example in the medical sector, where the gas flow must be clean. Also known are electrodynamic gas flow generators.

US 2019/242409 discloses a propulsion unit based on a ionic wind generator and a coanda device.

US745640 shows a cooler using an ionic wind generator. It does not rely on the coanda effect.

WO2011092755 shows an ozone generator having a needle electrode. Also this device does not work according to the coanda principle.

It is an object of the present invention to obviate the disadvantages of the prior art and in particular to provide an electrohydrodynamic gas flowrate amplifier for generating a gasflow and a corresponding method which is reliable, scalable and produces a clean gasflow.

The object is achieved by an electrohydrodynamic gas flowrate amplifier, a washer comprising an electrohydrodynamic gas flowrate amplifier, a method for amplifying gas and the use of an electrohydrodynamic gas flowrate amplifier according to the independent claims.

In particular this object is achieved by an electrohydrodynamic gas flowrate amplifier for generating a gasflow comprising a gasflow generator and a gas flow accelerator. A first gasflow is generable by the gasflow generator and directable to the gasflow accelerator. The gasflow accelerator is constructed in a way that a second gasflow is accelerable by the first gasflow. The gasflow accelerator is a Coanda gas flow accelerator employing the Coanda effect to accelerate the second gasflow by first gasflow from the gasflow generator to an gas outlet. The gasflow generator is an electro-hydrodynamic gas flow generator.

Such an electrohydrodynamic gas flowrate amplifier is reliable, silent and clean. An electro-hydrodynamic gas flow generator is advantageous in many application cases. An electro-hydrodynamic gas flow generator can be applied for cooling, noiseless fans, ionic pumps and plasma actuators, solid-state, bladeless fans without moving parts, propulsion engines, active flow control, surface drying, extraction of gases and small particles, decontamination and/or disinfection control. Disinfection control is advantageous to medical and food processing purposes as electrohydrodynamic gas flow generators produce ozone as a co-product. It is also possible, that an electro-hydrodynamic gas flow generator comprises a device for remove or dilute ozone to a subcritical level. In this case, the electro-hydrodynamic gas flow generator can be also used for domestic use.

The gas of the first gas flow can be any gas which can be ionized. The gas of the second gas flow can be any gas. Gas regarding to the invention can also be a gas mixture like air. The gas of the first gas flow and the gas of the second gas flow can be the same gas or a different gas. The gas of the first gas flow and/or the second gas flow can be air. In this case, the electrohydrodynamic gas flowrate amplifier is an air amplifier. The first gas flow from the gasflow generator is directed into the electrohydrodynamic gas flowrate amplifier in a way, that the gasflow from the first gasflow generator flows at the inner surface of the electrohydrodynamic gas flowrate amplifier. The first gas flow from the gas flow generator can be directed into the electrohydrodynamic gas flowrate amplifier in general parallel to the inner surface of the electrohydrodynamic gas flowrate amplifier. The electrohydrodynamic gas flowrate amplifier can be formed in such a way that the cross section increases in the direction of the first gas flow. Due to the direction of the first gas flow and the shape of the electrohydrodynamic gas flowrate amplifier, the fist gas flow from the gasflow generator is directed in the electrohydrodynamic gas flowrate amplifier near to the inner surface of the electrohydrodynamic gas flowrate amplifier in general in a direction parallel to the inner surface of the electrohydrodynamic gas flowrate amplifier. In this way the first gas flow can amplify the second gas flow in the gas flow accelerator. The gas flow accelerator can be at least partially curved. The gas flow accelerator can comprise an at least a partially hollow main body enclosing an inner area. The main body can have at least one outlet gas opening. The outlet gas opening is arranged on an inner surface of the main body. The inner surface faces the inner area. The outlet gas opening is directed such that gas is led in a direction substantially parallel to the inner surfaces. The main body comprises an gas inlet. The electro-hydrodynamic gas flow generator is connected to the gas inlet to create the first gas flow to the main body.

Such an electrohydrodynamic gas flowrate amplifier efficiently creates a clean gas flow.

The main body can be partially hollow or it can be fully hollow. The outlet gas opening can be at least partially curved. The outlet gas opening can comprise one or more openings. The outlet gas openings can have a circular shape. It can also be shaped like a square or oval. The gas inlet can have a circular, semicircular and/or elongated shape. The gas inlet can comprise several openings which can have the same or different shapes. The electrohydrodynamic gas flowrate amplifier can be made from metal, plastic or other material or a combination of different materials.

The electrohydrodynamic gas flowrate amplifier can comprise at least one of PA-GF, copper, and tungsten. PA-GF is a mouldable composite material comprising glass fibers in a matrix of a polymer material. The electrohydrodynamic gas flowrate amplifier can also comprise a combination of PA-GF, copper and tungsten. The electrohydrodynamic gas flowrate amplifier can be made out of a combination of PA-GF, copper, and tungsten.

The electrohydrodynamic gas flowrate amplifier can comprise a dielectric material. The electrohydrodynamic gas flowrate amplifier can comprise a 3D-printable material.

The electro-hydrodynamic gas flow generator can be designed as a corona discharge gas flow generator.

Such a corona discharge gas flow generator is easy to produce and scale and is reliable. It is also possible, that the electro-hydrodynamic gas flow generator is designed as a dielectric barrier discharge gas flow generator.

The electro-hydrodynamic gas flow generator according to the invention comprises one or more needle electrodes.

Needle electrodes have the advantage of generating a gas flow very well. Needle electrodes outperform wires in terms of discharge stability. Needle electrodes also outperform wires when a nearby dielectric is introduced. Therefore, it is very advantageous to arrange needle electrodes in the electro-hydrodynamic gas flow generator.

An electrohydrodynamic gas flowrate amplifier can comprise several units of needle electrodes, each unit comprising one, two or four needle electrodes. It is possible to arrange multiple electrohydrodynamic gas flowrate amplifiers in series. It is also possible to arrange electrohydrodynamic gas flowrate amplifier parallel. It is also possible to expand the cross-section of the electrohydrodynamic gas flowrate amplifier. Therefore, the design of the electrohydrodynamic gas flowrate amplifier is scalable.

The needle electrodes can be made from an electrically conducting material such as metal or metal alloy, in particular copper or tungsten.

The electro-hydrodynamic gas flow generator of the electrohydrodynamic gas flowrate amplifier can comprise a wire electrode.

A wire electrode can be formed very easily when the electrohydrodynamic gas flowrate amplifier is designed circular like a cylinder or a ring.

The electro-hydrodynamic gas flow generator can comprise one or more wire electrodes. It is also possible, that the electrohydrodynamic gas flow generator comprises needle electrodes and/or wire electrodes or even other electrodes or combinations of electrodes.

The electrohydrodynamic gas flowrate amplifier can comprise a collector. A collector is a grounded element which can conduct electric current. In the electrohydrodynamic gas flowrate amplifier, an ionic wind is generated between the electrodes and the collector. The electrodes can essentially be made out of tungsten. The collectors can be made out of copper. The collectors can be designed as plate collectors or as rod collectors.

The rod collector can be embedded in PA-GF material for the most part. The PA-GF material can be laser-sintered.

The distance between the electrodes and the collectors can be 20 mm to 30 mm. Preferably, the distance between the electrodes and the collectors is essentially 30 mm. The electrodes can be subjected to a voltage of 15 - 20 kV. When the electrohydrodynamic gas flowrate amplifier is operated with 15 kV to 17.5 kV, the electrohydrodynamic gas flowrate amplifier can be equipped with rod collectors. When the electrohydrodynamic gas flowrate amplifier is operated with 17.5 kV to 20 kV, the electrohydrodynamic gas flowrate amplifier can be equipped with plate collectors.

The inner surface of the electrohydrodynamic gas flowrate amplifier can have a gas entry side and an gas exit side. The outlet gas opening can be arranged substantially on the gas entry side. A surface enclosed by the gas entry side can be smaller than a surface enclosed by the gas exit side.

In this way the electrohydrodynamic gas flowrate amplifier can create an efficient acceleration.

The gas flow accelerator of the electrohydrodynamic gas flowrate amplifier can comprise a Coanda surface. The Coanda surface can be basically flat or convex shaped in the direction of the first gas flow. The Coanda surface can also be partly curved in a direction essentially perpendicular to the direction of the first gas flow. Preferably, the Coanda surface is formed like the inside of a cylinder.

With the Coanda effects and the Coanda surface the second gas flow can be amplified by the first gas flow effectively.

The gas flow accelerator can be formed essentially like a cylinder. In this case, the Coanda surface is shaped convex in the direction if the first gas flow and curved in a direction essentially perpendicular to the direction of the first gas flow. In this embodiment, the gas flow accelerator can advantageously be used in open space.

It is also possible that the Coanda surface is basically flat in the direction essentially perpendicular to the direction of the first gas flow. In this case, the gas flow accelerator can be formed essentially like a flat cuboid. In this embodiment, the gas flow accelerator can advantageously be used in closed space, for example in the housing of a washing or sterilizing machine for medical equipment.

A cross section of the inner surface can be at least partly shaped in a convex way.

With a convex cross section of the inner surface, the second gas stream can be amplified very well.

The object of the invention is also achieved by a washer for washing medical instruments comprising a washing chamber and an electrohydrodynamic gas flowrate amplifier as described before.

Such an electrohydrodynamic gas flowrate amplifier can produce a clean gas flow. For this, it is advantages to arrange such an electrohydrodynamic gas flowrate amplifier to a washer washing for medical instruments. There can be one or more electrohydrodynamic gas flowrate amplifiers be arranged to the washing chamber. In particular, as during the electro hydrodynamic gas-flow generation ozone is also generated the gas created by the electrohydrodynamic gas flowrate amplifier automatically disinfects the area to be dried.

The washer for medical instruments can be built in a way that the outlet of the electrohydrodynamic gas flowrate amplifier is directed into the washing chamber washing chamber such that the electrohydrodynamic gas flowrate amplifier enhances a drying process in the washer.

The electrohydrodynamic gas flowrate amplifier can be arranged above the washing chamber. The electrohydrodynamic gas flowrate amplifier can also be arranged at a side of the washing chamber or below the washing chamber. It is also possible that the outlets of a plurality of electrohydrodynamic gas flowrate amplifiers are connected to the washing chamber enhancing a drying process in the washer. The outlets of the plurality of electrohydrodynamic gas flowrate amplifiers can be connected to the bottom of the washing chamber and/or to the side of the chamber and/or to the top of the chamber.

The object of the invention is also achieved by a sterilizer for sterilizing medical instruments comprising a sterilizing chamber and an electrohydrodynamic gas flowrate amplifier as described before.

Such an electrohydrodynamic gas flowrate amplifier can produce a clean gas flow. For this, it is advantages to arrange such an electrohydrodynamic gas flowrate amplifier to a sterilizer for sterilizing medical instruments. There can be one or more electrohydrodynamic gas flowrate amplifiers be arranged to the washing chamber. In particular, during the electro hydrodynamic gasflow generation ozone is also generated the gas created by the electrohydrodynamic gas flowrate amplifier automatically, which is advantageous for sterilizing medical instruments.

The sterilizer for medical instruments can be built in a way that the outlet of the electrohydrodynamic gas flowrate amplifier is directed into the sterilizing chamber such that the electrohydrodynamic gas flowrate amplifier enhances a drying and/or sterilization process in the washer.

The electrohydrodynamic gas flowrate amplifier can be arranged above the sterilizing chamber. The electrohydrodynamic gas flowrate amplifier can also be arranged at a side of the sterilizing chamber or below the sterilizing chamber. It is also possible that the outlets of a plurality of electrohydrodynamic gas flowrate amplifiers are connected to the sterilizing chamber enhancing a drying and/or sterilizing process in the sterilizer. The outlets of the plurality of electrohydrodynamic gas flowrate amplifiers can be connected to the bottom of the sterilizing chamber and/or to the side of the chamber and/or to the top of the chamber.

The object of the invention is also achieved by a method of amplifying gas using an electrohydrodynamic gas flowrate amplifier as described before. In the method a first gas flow is generated by the gas flow generator and a second gas flow is amplified by the first gas flow in the gas flow accelerator.

Such a method is reliable and efficient.

In the method the gas flow generator can be an electrohydrodynamic generator generating an ionic wind.

In the method, a first gas flow can be generated by the gas flow generator. The first gas flow can be directed to the gas flow accelerator. A second gas flow can be amplified by the first gas flow.

The object of the invention is also achieved by the use of an electrohydrodynamic gas flowrate amplifier as described before for drying. The electrohydrodynamic gas flowrate amplifier can be used for drying in a medical washer.

A medical washer is a washer for medical equipment such as surgical instruments or other medical material.

A sterilizer is a sterilizer for sterilizing and/or disinfecting medical equipment such as surgical instruments or other medical material.

It is also possible, that the medical equipment is washed and sterilized and/or disinfected in the same machine. In this case, the machine is a medical washer and a sterilizer.

The electrohydrodynamic gas flowrate amplifier as described before can also be used for disinfection.

The electrohydrodynamic gas flowrate amplifier can also be used for other applications where airflow generation is required. It can replace mechanical fans in various applications, for example in building ventilation or in household appliances like hairdryers or dishwashers.

The invention is further described in embodiments by means of figures in the following:
- Figure 1:: A longitudinal cross-section through an electrohydrodynamic gas flowrate amplifier;
- Figure 2:: an electrohydrodynamic gas flowrate amplifier with a gas entry side and a gas exit side;
- Figure 3:: a detail of an electrohydrodynamic gas flowrate amplifier with a first gas flow, a second gas flow and a Coanda surface;
- Figure 4:: an electrohydrodynamic gas flowrate amplifier with a first gas flow, a second gas flow and a needle electrode;
- Figure 5:: a gas flowrate amplifier operating with pressurized gas as known in the prior art wherein the gas entry side has a larger outer diameter than the gas exit side;
- Figure 6:: a longitudinal section of a gas flowrate amplifier operating with pressurized gas as known in the prior art wherein the gas entry side has a larger diameter than the gas exit side;
- Figure 7:: a longitudinal cross-section through an electrohydrodynamic gas flowrate amplifier with needle electrodes;
- Figure 8:: a longitudinal cross-section through three electrohydrodynamic gas flowrate amplifier with needle electrodes which are attached one after another;
- Figure 9:: a longitudinal cross-section through an electrohydrodynamic gas flowrate amplifier with needle electrodes, the inner surface being convex,
- Figure 10:: a longitudinal cross-section trough an electrohydrodynamic gas flowrate amplifier with plate collectors,
- Figure 11:: a longitudinal cross-section trough an electrohydrodynamic gas flowrate amplifier with rod collectors,
- Figure 12:: a needle electrode.

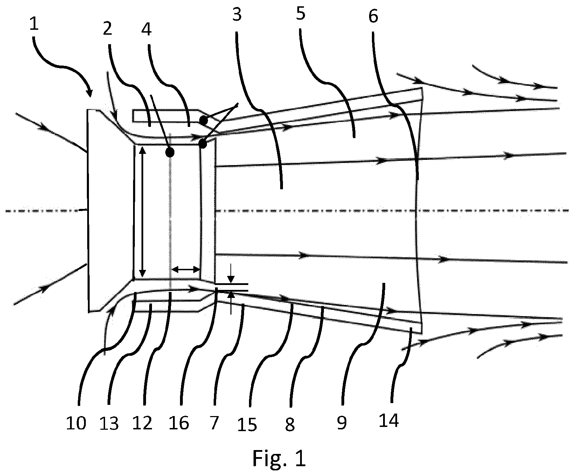

Figure 1 shows a longitudinal cross-section through an electrohydrodynamic gas flowrate amplifier 1. The electrohydrodynamic gas flowrate amplifier 1 comprises a gas flow generator 4 which generates a first gas flow 2. The electrohydrodynamic gas flowrate amplifier 1 also comprises a gas flow accelerator 5 where the second gas flow 3 is amplified by the first gas flow 2. The second gas flow 3 exits the electrohydrodynamic gas flowrate amplifier 1 at the gas outlet 6. The electrohydrodynamic gas flowrate amplifier has a main body 7. The electrohydrodynamic gas flowrate amplifier comprises an inner surface 8. The inner area 9 of the main body 7 is shaped as a truncated cone. The electrohydrodynamic gas flowrate amplifier 1 comprises an gas inlet 10 which is ring-shaped. The gas flow generator 4 comprises a wire electrode 12. At the wire electrode 12 the first gas flow 2 is created. The electrohydrodynamic gas flowrate amplifier 1 has a gas outlet opening 16. The gas outlet opening 16 is ring-shaped. The electrohydrodynamic gas flowrate amplifier 1 has an gas entry side 13 and an gas exit side 14. The outlet gas opening 16 is arranged on an inner surface 8 of the main body 7. The inner surface 8 faces the inner area 9. The outlet gas opening 16 directs the first gas flow 2 in a direction substantially parallel to the inner surface 8. The electrohydrodynamic gas flowrate amplifier 1 comprises a Coanda surface 15 which is arranged on the inner surface 8 of the electrohydrodynamic gas flowrate amplifier 1.

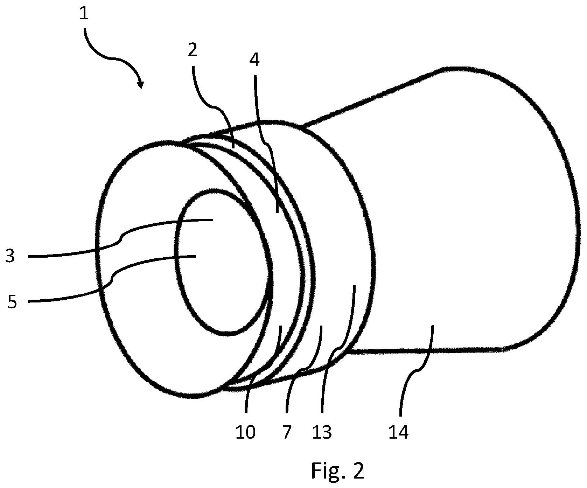

Figure 2 shows an amplifier 1 with a gas entry side 13 and a gas exit side 14. The electrohydrodynamic gas flowrate amplifier 1 comprises a gas flow generator 4 where the first gas flow 2 is generated. The electrohydrodynamic gas flowrate amplifier 1 also comprises an gas flow accelerator 5 where a second gas flow 3 is generated. The main body 7 of the electrohydrodynamic gas flowrate amplifier 1 is formed in a cylindrically. The electrohydrodynamic gas flowrate amplifier 1 has a gas inlet 10 which is ring-shaped.

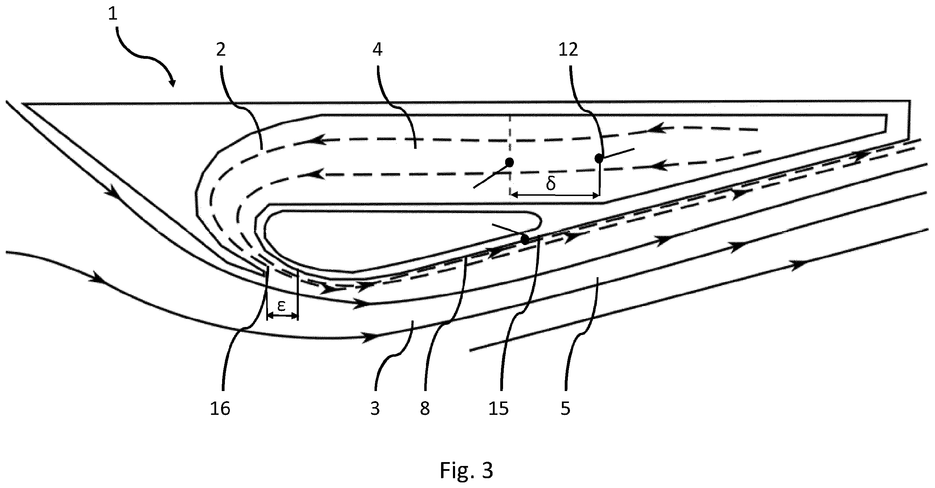

Figure 3 shows a detail of an electrohydrodynamic gas flowrate amplifier 1 with a first gas flow 2, a second gas flow 3 and a Coanda surface 15. The electrohydrodynamic gas flowrate amplifier 1 also comprises a gas flow accelerator 5 accelerating a second gas flow 3. The electrohydrodynamic gas flowrate amplifier 1 has a gas outlet opening 16 where the first gas flow 2 from the gas flow generator 4 meets the second gas flow 3 of the gas flow accelerator 5. The inner surface 8 of the electrohydrodynamic gas flowrate amplifier 1 comprises a Coanda surface 15. The gas flow generator 4 of the electrohydrodynamic gas flowrate amplifier 1 has a wire electrode 12.

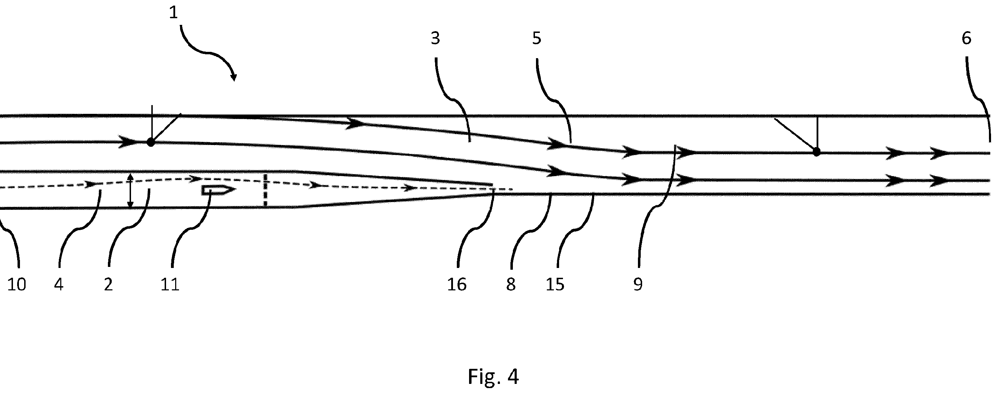

Figure 4 shows an electrohydrodynamic gas flowrate amplifier 1 with a first gas flow 2, a second gas flow 3 and a needle electrode 11. The electrohydrodynamic gas flowrate amplifier 1 comprises a gas flow generator 4 which generates a first gas flow 2. The electrohydrodynamic gas flowrate amplifier 1 also comprises a gas flow accelerator 5, where the second gas flow 3 is amplified by the first gas flow 2. The gas flow generator 4 comprises a needle electrode 11. The needle electrode 11 generates the first gas flow 2. The gas enters the gas flow generator 4 of the electrohydrodynamic gas flowrate amplifier 1 through a gas inlet 10. The inner surface 8 of the electrohydrodynamic gas flowrate amplifier 1 comprises a Coanda surface 15. The first gas flow 2 and the second gas flow 3 are both in the inner area 9 of the electrohydrodynamic gas flowrate amplifier 1. The second gas flow 3 escapes from the electrohydrodynamic gas flowrate amplifier 1 through the gas outlet 6.

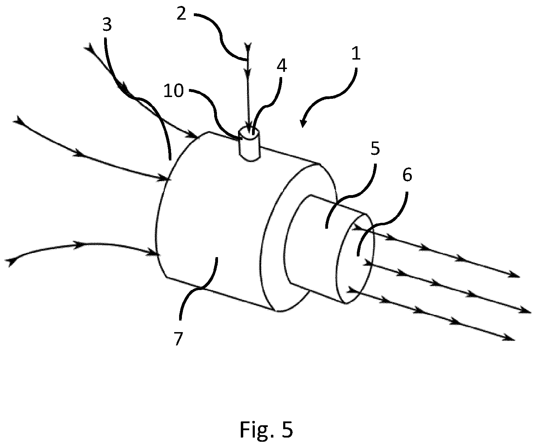

Figure 5 shows a gas flowrate amplifier 1 operating with pressurized gas as known in the prior art wherein the gas entry side 13 has a larger outer diameter than the gas exit side 14. The gas flowrate amplifier 1 is fed with pressurized gas by the first gas flow 2. The gas flowrate amplifier 1 comprises a gas flow generator 4, a gas flow accelerator 5 and a main body 7. The first gas flow 2 enters the gas flowrate amplifier 1 through the gas inlet 10. The second gas flow 9 flows to the main body 7 of the gas flowrate amplifier 1. The first gas flow 2 and the second gas flow 3 meet in the main body 7 of the as flowrate amplifier 1. The first gas flow 2 and the second gas flow 3 leave the gas flowrate amplifier 1 through the gas outlet 6.

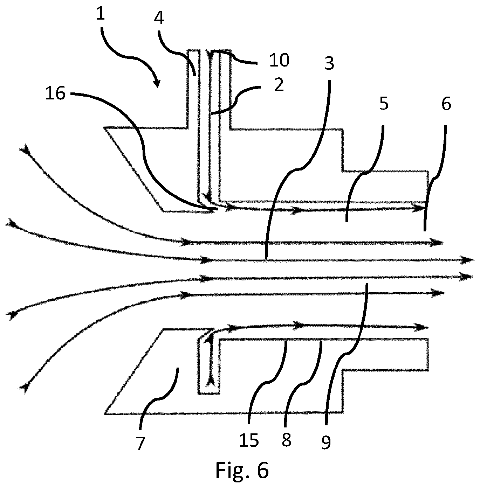

Figure 6 shows a longitudinal section of a gas flowrate amplifier 1 operating with pressurized gas as known in the prior art wherein the gas entry side 13 has a larger outer diameter than the gas exit side 14. The gas flowrate amplifier 1 is fed with pressurized gas by the first gas flow 2. The gas flowrate amplifier 1 comprises a gas flow generator 4 and a gas flow accelerator 5. The first gas flow 2 is generated by the gas flow generator 4. The second gas flow flows through the gas flow accelerator 5. The second gasflow 3 is accelerated by the first gas flow 2. The first gas flow 2 enters the gas flow accelerator 5 through a gas outlet opening 16. The main body 7 is formed cylindrical. The inner area 9 of the main body 7 of the gas flowrate amplifier 1 comprises an inner surface 8. The inner surface 8 comprises a Coanda surface 15. The first gas flow 2 and the second gas flow 3 leave the gas flowrate amplifier 1 through the gas outlet 6.

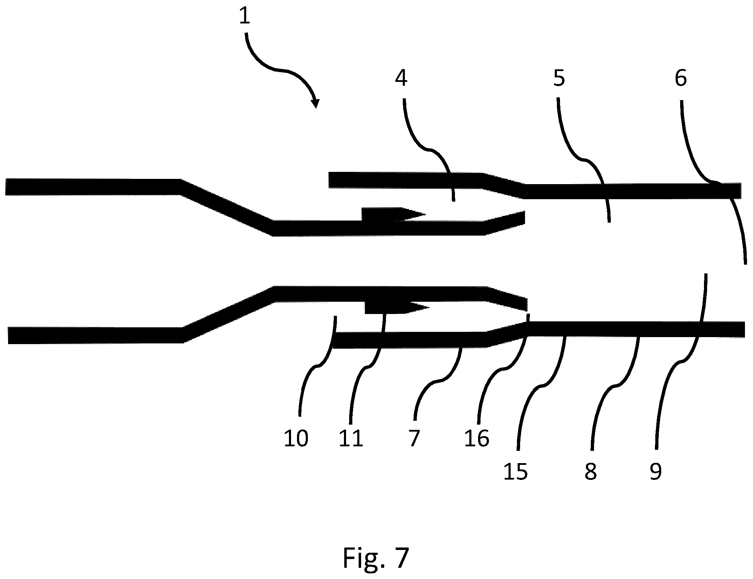

Figure 7 shows a longitudinal cross-section through an electrohydrodynamic gas flowrate amplifier 1 with needle electrodes 11. The electrohydrodynamic gas flowrate amplifier 1 comprises a gas flow generator 4 and a gas flow accelerator 5. The electrohydrodynamic gas flowrate amplifier has a main body 7. The electrohydrodynamic gas flowrate amplifier comprises an inner surface 8. The electrohydrodynamic gas flowrate amplifier 1 comprises a gas inlet 10 which is ring-shaped. The gas flow generator 4 comprises needle electrodes 11. The electrohydrodynamic gas flowrate amplifier 1 has a gas outlet opening 16. The gas outlet opening 16 is ring-shaped. The outlet gas opening 16 is arranged on an inner surface 8 of the main body 7. The inner surface 8 faces the inner area 9. The outlet gas opening 16 directs the first gas flow 2 in a direction substantially parallel to the inner surface 8. The electrohydrodynamic gas flowrate amplifier 1 comprises a Coanda surface 15 which is arranged on the inner surface 8 of the electrohydrodynamic gas flowrate amplifier 1.

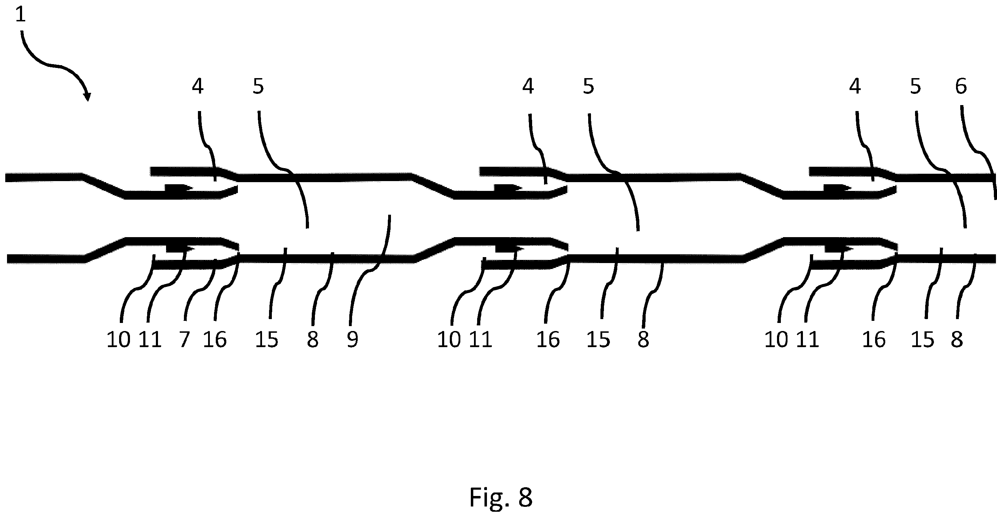

Figure 8 shows a longitudinal cross-section through three electrohydrodynamic gas flowrate amplifiers 1 with needle electrodes 11. The three electrohydrodynamic gas flowrate amplifiers 1 are attached one after another. Each electrohydrodynamic gas flowrate amplifier 1 comprises a unit of needle electrodes 11. Each unit comprises two needle electrodes 11. It is possible that a unit comprises one, two or four needle electrodes. Identical reference signs indicate the same components.

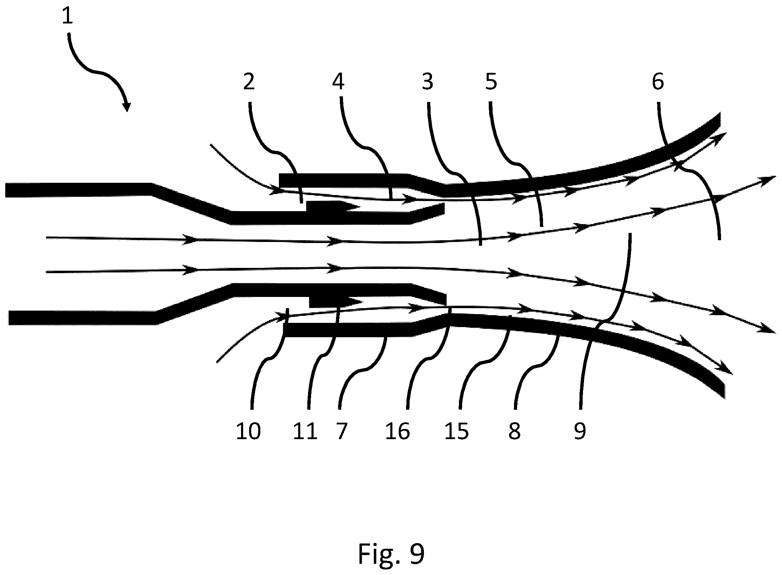

Figure 9 shows a longitudinal cross-section through an electrohydrodynamic gas flowrate amplifier 1 with needle electrodes 11, the inner surface 8 being convex. At the needle electrodes 11, the first gas flow 2 is created. The first gas flow 2 enters the inner area 9 through the gas outlet opening 16. The first gas flow 2 amplifies the second gas flow 3 in the inner area 9. Identical reference signs indicate the same components,

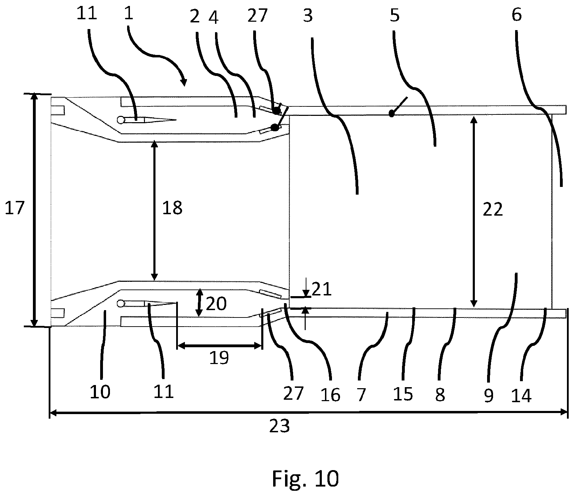

Figure 10 shows a longitudinal cross-section trough an electrohydrodynamic gas flowrate amplifier 1 analogously to Figure 1. In contrast to the gas flowrate amplifier in figure 1, the gas flowrate amplifier in figure 10 has needle electrodes 11. Furthermore, the gas flowrate amplifier 1 of figure 10 is equipped with plate collectors 27. The main body 7 is essentially shaped as a cylinder. The outer diameter 17 of the gas flowrate amplifier 1 is 82 mm. The first inner diameter 18 of the gas flow accelerator 5 is 50 mm. The second inner diameter 22 of the gas flow accelerator 5 is 70 mm. The second inner diameter 22 is arranged downstream of the first inner diameter 18. The clear width 20 of the gas flow generator 4 is 10 mm. The clear width 21 of the gas outlet opening 16 is 4 mm. The distance 19 between the needle electrodes 11 and the plate collectors 27 is 30 mm. The length 23 of the gas flowrate amplifier 1 is 179 mm.

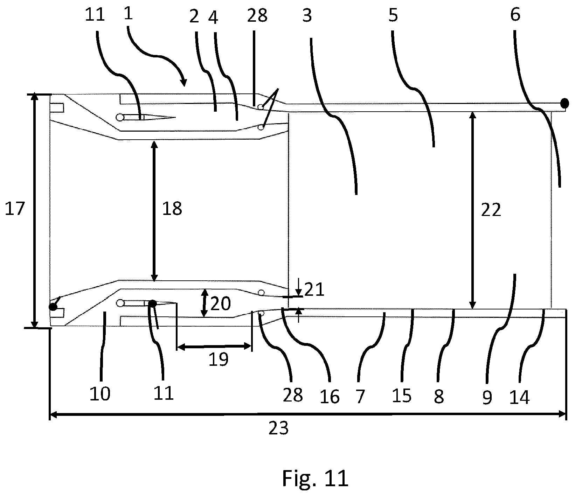

Figure 11 shows a longitudinal cross-section trough an electrohydrodynamic gas flowrate amplifier 1 analogously to figure 10. In contrast to the gas flowrate amplifier 1 of figure 10, the gas flowrate amplifier 1 in figure 11 is equipped with rod collectors 28.

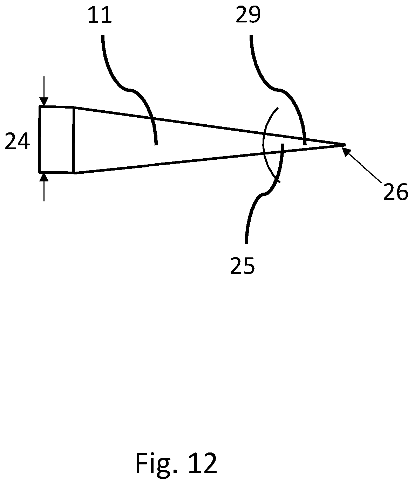

Figure 12 shows a needle electrode 11. The needle electrode 11 has a maximum needle diameter 24 of 2mm. The angle 25 of the needle tip 29 of the needle electrode 11 is 10°. The radius 26 of the needle tip 29 is 100 µm.

## Claims

1. Electrohydrodynamic gas flowrate amplifier (1) for generating a gas flow comprising a gasflow generator (4) and a gasflow accelerator (5), wherein a first gas flow (2) is generable by the gas flow generator (4) and directable to the gasflow accelerator (5) and the gasflow accelerator (5) is constructed in a way that a second gas flow (3) is accelerable by the first gas flow (2), wherein the gasflow accelerator (5) is a Coanda gas flow accelerator employing the Coanda effect to accelerate the second gas flow (3) by the first gas flow (2) from the gasflow generator (4) to an gas outlet (6), wherein the gas flow generator is an electro-hydrodynamic gas flow generator (4), **characterized in that** the gasflow generator (4) comprises at least one, in particular two or four, needle electrodes (11).

2. Electrohydrodynamic gas flowrate amplifier (1) according to claim 1, **characterized in that** the electro-hydrodynamic gas flow generator is a corona discharge gas flow generator or a dielectric barrier discharge gas flow generator.

3. Electrohydrodynamic gas flowrate amplifier (1) according to any one of the preceding claims, **characterized in that** the inner surface (8) has an gas entry side (13) and a gas exit side (14), wherein the outlet gas opening (16) is arranged substantially on the gas entry side (13) and a surface enclosed by the gas entry side (13) is smaller than a surface enclosed by the gas exit side (14).

4. Electrohydrodynamic gas flowrate amplifier (1) according to any one of the preceding claims, **characterized in that** the gas flow accelerator (5) comprises a Coanda surface, wherein the Coanda surface is basically flat or convex-shaped in the direction of the first gas flow and wherein the Coanda surface is preferably partly curved in a direction essentially perpendicular to the direction of the first gas flow.

5. Electrohydrodynamic gas flowrate amplifier (1) according to any one of the preceding claims, **characterized in that** a cross section of the inner surface (8) is at least partly shaped in a convex way.

6. Washer for washing medical instruments comprising a washing chamber and an electrohydrodynamic gas flowrate amplifier for generating a gas flow comprising a gasflow generator (4) and a gasflow accelerator (5), wherein a first gas flow (2) is generable by the gas flow generator (4) and directable to the gasflow accelerator (5) and the gasflow accelerator (5) is constructed in a way that a second gas flow (3) is accelerable by the first gas flow (2), wherein the gasflow accelerator (5) is a Coanda gas flow accelerator employing the Coanda effect to accelerate the second gas flow (3) by the first gas flow (2) from the gasflow generator (4) to an gas outlet (6), wherein the gas flow generator is an electro-hydrodynamic gas flow generator (4).

7. Washer according to claim 6, **characterized in that** the outlet (6) of the electrohydrodynamic gas flowrate amplifier (1) is directed into the washing chamber such that the electro- hydrodynamic gas flowrate amplifier (1) enhances a drying process in the washer.

8. Sterilizer for sterilizing medical instruments comprising a sterilizing chamber and an electrohydrodynamic gas flowrate amplifier (1) for generating a gas flow comprising a gasflow generator (4) and a gasflow accelerator (5), wherein a first gas flow (2) is generable by the gas flow generator (4) and directable to the gasflow accelerator (5) and the gasflow accelerator (5) is constructed in a way that a second gas flow (3) is accelerable by the first gas flow (2), wherein the gasflow accelerator (5) is a Coanda gas flow accelerator employing the Coanda effect to accelerate the second gas flow (3) by the first gas flow (2) from the gasflow generator (4) to an gas outlet (6), wherein the gas flow generator is an electrohydrodynamic gas flow generator (4).

9. Sterilizer according to claim 8, **characterized in that** the outlet (6) of the electrohydrodynamic gas flowrate amplifier (1) is directed into the sterilizing chamber such that the electrohydrodynamic gas flowrate amplifier (1) enhances a sterilizing and/or drying process in the sterilizer.

10. Method of amplifying gas using an electrohydrodynamic gas flowrate amplifier (1) according to any one of claims 1 to 5, **characterized in that** a first gas flow (2) is generated by the gas flow generator (4) and a second gas flow (3) is amplified by the first gas flow (2) in the gas flow accelerator (5).

11. Method according to claim 10, **characterized in that** the gas flow generator (4) is an electro-hydrodynamic generator generating an ionic wind.

12. Method according to any one of claims 10 or 11, **characterized in that** the first gas flow (2) generated by the gas flow generator (4) is directed to the gas flow accelerator (5), wherein the second gas flow (3) is accelerated in the gas flow accelerator (5) using the Coanda effect.

13. Use of an electrohydrodynamic gas flowrate amplifier (1) according to any one of claims 1 to 5 for amplifying gas, in particular in a medical washer.

14. Use of an electrohydrodynamic gas flowrate amplifier (1) for disinfection, the electrohydrodynamic gas flowrate amplifier (1) being for generating a gas flow comprising a gasflow generator (4) and a gasflow accelerator (5), wherein a first gas flow (2) is generable by the gas flow generator (4) and directable to the gasflow accelerator (5) and the gasflow accelerator (5) is constructed in a way that a second gas flow (3) is accelerable by the first gas flow (2), wherein the gasflow accelerator (5) is a Coanda gas flow accelerator employing the Coanda effect to accelerate the second gas flow (3) by the first gas flow (2) from the gasflow generator (4) to an gas outlet (6), wherein the gas flow generator is an electro-hydrodynamic gas flow generator (4).

## Patentansprüche

1. Elektrohydrodynamischer Gasströmungsverstärker (1) zum Erzeugen eines Gasstroms, aufweisend einen Gasstromgenerator (4) und einen Gasstrombeschleuniger (5), wobei ein erster Gasstrom (2) durch den Gasstromgenerator (4) erzeugt werden kann und zu dem Gasstrombeschleuniger (5) geleitet werden kann und der Gasstrombeschleuniger (5) so konstruiert ist, dass ein zweiter Gasstrom (3) durch den ersten Gasstrom (2) beschleunigt werden kann, wobei der Gasstrombeschleuniger (5) ein Coanda-Gasstrombeschleuniger ist, der den Coanda-Effekt nutzt, um den zweiten Gasstrom (3) durch den ersten Gasstrom (2) von dem Gasstromgenerator (4) zu einem Gasauslass (6) zu beschleunigen, wobei der Gasstromgenerator ein elektrohydrodynamischer Gasstromgenerator (4) ist, **dadurch gekennzeichnet, dass** der Gasstromgenerator (4) mindestens eine, insbesondere zwei oder vier, Nadelelektroden (11) aufweist.

2. Elektrohydrodynamischer Gasströmungsverstärker (1) gemäß Anspruch 1, **dadurch gekennzeichnet, dass** der elektrohydrodynamische Gasstromgenerator ein Korona-Entladung-Gasstromgenerator oder ein Dielektrische-Barriereentladung-Gasstromgenerator ist.

3. Elektrohydrodynamischer Gasströmungsverstärker (1) gemäß einem der vorstehenden Ansprüche, **dadurch gekennzeichnet, dass** die Innenfläche (8) eine Gaseintrittsseite (13) und eine Gasaustrittsseite (14) aufweist, wobei die Gasauslassöffnung (16) im Wesentlichen an der Gaseintrittsseite (13) angeordnet ist und eine von der Gaseintrittsseite (13) umschlossene Fläche kleiner als eine von der Gasaustrittsseite (14) umschlossene Fläche ist.

4. Elektrohydrodynamischer Gasströmungsverstärker (1) gemäß einem der vorstehenden Ansprüche, **dadurch gekennzeichnet, dass** der Gasstrombeschleuniger (5) eine Coanda-Oberfläche aufweist, wobei die Coanda-Oberfläche in der Richtung des ersten Gasstroms im Wesentlichen flach oder konvex geformt ist und wobei die Coanda-Oberfläche vorzugsweise teilweise in einer Richtung gekrümmt ist, die im Wesentlichen senkrecht zu der Richtung des ersten Gasstroms ist.

5. Elektrohydrodynamischer Gasströmungsverstärker (1) gemäß einem der vorstehenden Ansprüche, **dadurch gekennzeichnet, dass** ein Querschnitt der Innenfläche (8) zumindest teilweise konvex geformt ist.

6. Wäscher zum Waschen medizinischer Instrumente, aufweisend eine Waschkammer und einen elektrohydrodynamischen Gasströmungsverstärker zum Erzeugen eines Gasstroms, aufweisend einen Gasstromgenerator (4) und einen Gasstrombeschleuniger (5), wobei ein erster Gasstrom (2) durch den Gasstromgenerator (4) erzeugt werden kann und zu dem Gasstrombeschleuniger (5) geleitet werden kann und der Gasstrombeschleuniger (5) so konstruiert ist, dass ein zweiter Gasstrom (3) durch den ersten Gasstrom (2) beschleunigt werden kann, wobei der Gasstrombeschleuniger (5) ein Coanda-Gasstrombeschleuniger ist, der den Coanda-Effekt nutzt, um den zweiten Gasstrom (3) durch den ersten Gasstrom (2) von dem Gasstromgenerator (4) zu einem Gasauslass (6) zu beschleunigen, wobei der Gasstromgenerator ein elektrohydrodynamischer Gasstromgenerator (4) ist.

7. Wäscher gemäß Anspruch 6, **dadurch gekennzeichnet, dass** der Auslass (6) des elektrohydrodynamischen Gasströmungsverstärkers (1) in die Waschkammer gerichtet ist, so dass der elektrohydrodynamische Gasströmungsverstärker (1) einen Trocknungsprozess in dem Wäscher verbessert.

8. Sterilisator zum Sterilisieren medizinischer Instrumente, aufweisend eine Sterilisationskammer und einen elektrohydrodynamischen Gasströmungsverstärker (1) zum Erzeugen eines Gasstroms, aufweisend einen Gasstromgenerator (4) und einen Gasstrombeschleuniger (5), wobei ein erster Gasstrom (2) durch den Gasstromgenerator (4) erzeugt werden kann und zu dem Gasstrombeschleuniger (5) geleitet werden kann und der Gasstrombeschleuniger (5) so konstruiert ist, dass ein zweiter Gasstrom (3) durch den ersten Gasstrom (2) beschleunigt werden kann, wobei der Gasstrombeschleuniger (5) ein Coanda-Gasstrombeschleuniger ist, der den Coanda-Effekt nutzt, um den zweiten Gasstrom (3) durch den ersten Gasstrom (2) von dem Gasstromgenerator (4) zu einem Gasauslass (6) zu beschleunigen, wobei der Gasstromgenerator ein elektrohydrodynamischer Gasstromgenerator (4) ist.

9. Sterilisator gemäß Anspruch 8, **dadurch gekennzeichnet, dass** der Auslass (6) des elektrohydrodynamischen Gasströmungsverstärkers (1) in die Sterilisationskammer gerichtet ist, so dass der elektrohydrodynamische Gasströmungsverstärker (1) einen Sterilisationsund/oder Trocknungsprozess in dem Sterilisator verbessert.

10. Verfahren zum Verstärken von Gas unter Verwendung eines elektrohydrodynamischen Gasströmungsverstärkers (1) gemäß einem der Ansprüche 1 bis 5, **dadurch gekennzeichnet, dass** ein erster Gasstrom (2) durch den Gasstromgenerator (4) erzeugt wird und ein zweiter Gasstrom (3) durch den ersten Gasstrom (2) in dem Gasstrombeschleuniger (5) verstärkt wird.

11. Verfahren gemäß Anspruch 10, **dadurch gekennzeichnet, dass** der Gasstromgenerator (4) ein elektrohydrodynamischer Generator ist, der einen Ionenwind erzeugt.

12. Verfahren gemäß einem der Ansprüche 10 oder 11, **dadurch gekennzeichnet, dass** der von dem Gasstromgenerator (4) erzeugte erste Gasstrom (2) zu dem Gasstrombeschleuniger (5) geleitet wird, wobei der zweite Gasstrom (3) in dem Gasstrombeschleuniger (5) unter Verwendung des Coanda-Effekts beschleunigt wird.

13. Verwendung eines elektrohydrodynamischen Gasströmungsverstärkers (1) gemäß einem der Ansprüche 1 bis 5 zum Verstärken von Gas, insbesondere in einem medizinischen Wäscher.

14. Verwendung eines elektrohydrodynamischen Gasströmungsverstärkers (1) zur Desinfektion, wobei der elektrohydrodynamische Gasströmungsverstärker (1) zum Erzeugen eines Gasstroms einen Gasstromgenerator (4) und einen Gasstrombeschleuniger (5) aufweist, wobei ein erster Gasstrom (2) durch den Gasstromgenerator (4) erzeugt werden kann und zu dem Gasstrombeschleuniger (5) geleitet werden kann und der Gasstrombeschleuniger (5) so konstruiert ist, dass ein zweiter Gasstrom (3) durch den ersten Gasstrom (2) beschleunigt werden kann, wobei der Gasstrombeschleuniger (5) ein Coanda-Gasstrombeschleuniger ist, der den Coanda-Effekt nutzt, um den zweiten Gasstrom (3) durch den ersten Gasstrom (2) von dem Gasstromgenerator (4) zu einem Gasauslass (6) zu beschleunigen, wobei der Gasstromgenerator ein elektrohydrodynamischer Gasstromgenerator
(4) ist.

## Revendications

1. Amplificateur électrohydrodynamique de débit de gaz (1) pour générer un flux de gaz comprenant un générateur de flux de gaz (4) et un accélérateur de flux de gaz (5), dans lequel un premier flux de gaz (2) peut être généré par le générateur de flux de gaz (4) et peut être dirigé vers l'accélérateur de flux de gaz (5) et l'accélérateur de flux de gaz (5) est construit de sorte qu'un second flux de gaz (3) puisse être accéléré par le premier flux de gaz (2), dans lequel l'accélérateur de flux de gaz (5) est un accélérateur de flux de gaz Coanda utilisant l'effet Coanda pour accélérer le second flux de gaz (3) par le premier flux de gaz (2) du générateur de flux de gaz (4) vers une sortie de gaz (6), dans lequel le générateur de flux de gaz est un générateur électrohydrodynamique de flux de gaz (4), **caractérisé en ce que** le générateur de flux de gaz (4) comprend au moins une, en particulier deux ou quatre, aiguilles-électrodes (11).

2. Amplificateur électrohydrodynamique de débit de gaz (1) selon la revendication 1, **caractérisé en ce que** le générateur électrohydrodynamique de débit de gaz est un générateur de débit de gaz à décharge par effet couronne ou un générateur de débit de gaz à décharge à barrière diélectrique.

3. Amplificateur électrohydrodynamique de débit de gaz (1) selon l'une quelconque des revendications précédentes, **caractérisé en ce que** la surface intérieure (8) présente un côté entrée de gaz (13) et un côté sortie de gaz (14), dans lequel l'ouverture de gaz de sortie (16) est agencée sensiblement sur le côté entrée de gaz (13) et une surface entourée par le côté entrée de gaz (13) est plus petite qu'une surface entourée par le côté sortie de gaz (14).

4. Amplificateur électrohydrodynamique de débit de gaz (1) selon l'une quelconque des revendications précédentes, **caractérisé en ce que** l'accélérateur de flux de gaz (5) comprend une surface Coanda, dans lequel la surface Coanda est sensiblement plate ou convexe dans la direction du premier flux de gaz et dans lequel la surface Coanda est de préférence partiellement incurvée dans une direction sensiblement perpendiculaire à la direction du premier flux de gaz.

5. Amplificateur électrohydrodynamique de débit de gaz (1) selon l'une quelconque des revendications précédentes, **caractérisé en ce qu'**une section transversale de la surface interne (8) est au moins partiellement formée de manière convexe.

6. Dispositif de lavage pour laver des instruments médicaux comprenant une chambre de lavage et un amplificateur électrohydrodynamique de débit de gaz pour générer un flux de gaz comprenant un générateur de flux de gaz (4) et un accélérateur de flux de gaz (5), dans lequel un premier flux de gaz (2) peut être généré par le générateur de flux de gaz (4) et peut être dirigé vers l'accélérateur de flux de gaz (5) et l'accélérateur de flux de gaz (5) est construit de sorte qu'un second flux de gaz (3) puisse être accéléré par le premier flux de gaz (2), dans lequel l'accélérateur de flux de gaz (5) est un accélérateur de flux de gaz Coanda utilisant l'effet Coanda pour accélérer le second flux de gaz (3) par le premier flux de gaz (2) du générateur de flux de gaz (4) vers une sortie de gaz (6), dans lequel 1e générateur de flux de gaz est un générateur électrohydrodynamique de flux de gaz (4).

7. Dispositif de lavage selon la revendication 6, **caractérisé en ce que** la sortie (6) de l'amplificateur électrohydrodynamique de débit de gaz (1) est dirigée dans la chambre de lavage de sorte que l'amplificateur électrohydrodynamique de débit de gaz (1) améliore un processus de séchage dans le dispositif de lavage.

8. Stérilisateur pour stériliser des instruments médicaux comprenant une chambre de stérilisation et un amplificateur électrohydrodynamique de débit de gaz (1) pour générer un flux de gaz comprenant un générateur de flux de gaz (4) et un accélérateur de flux de gaz (5), dans lequel un premier flux de gaz (2) peut être généré par le générateur de flux de gaz (4) et peut être dirigé vers l'accélérateur de flux de gaz (5) et l'accélérateur de flux de gaz (5) est construit de sorte qu'un second flux de gaz (3) puisse être accéléré par le premier flux de gaz (2), dans lequel l'accélérateur de flux de gaz (5) est un accélérateur de flux de gaz Coanda utilisant l'effet Coanda pour accélérer le second flux de gaz (3) par le premier flux de gaz (2) du générateur de flux de gaz (4) vers une sortie de gaz (6), dans lequel 1e générateur de flux de gaz est un générateur électrohydrodynamique de flux de gaz (4).

9. Stérilisateur selon la revendication 8, **caractérisé en ce que** la sortie (6) de l'amplificateur électrohydrodynamique de débit de gaz (1) est dirigée dans la chambre de stérilisation de sorte que l'amplificateur électrohydrodynamique de débit de gaz (1) améliore un processus de stérilisation et/ou de séchage dans le stérilisateur.

10. Procédé d'amplification de gaz utilisant un amplificateur électrohydrodynamique de débit de gaz (1) selon l'une quelconque des revendications 1 à 5, **caractérisé en ce qu'**un premier flux de gaz (2) est généré par le générateur de flux de gaz (4) et un second flux de gaz (3) est amplifié par le premier flux de gaz (2) dans l'accélérateur de flux de gaz (5).

11. Procédé selon la revendication 10, **caractérisé en ce que** le générateur de flux de gaz (4) est un générateur électrohydrodynamique générant un vent ionique.

12. Procédé selon l'une quelconque des revendications 10 ou 11, **caractérisé en ce que** le premier flux de gaz (2) généré par le générateur de flux de gaz (4) est dirigé vers l'accélérateur de flux de gaz (5), dans lequel le second flux de gaz (3) est accéléré dans l'accélérateur de flux de gaz (5) en utilisant l'effet Coanda.

13. Utilisation d'un amplificateur électrohydrodynamique de débit de gaz (1) selon l'une quelconque des revendications 1 à 5 pour amplifier un gaz, en particulier dans un dispositif de lavage médical.

14. Utilisation d'un amplificateur électrohydrodynamique de débit de gaz (1) pour la désinfection, l'amplificateur électrohydrodynamique de débit de gaz (1) étant destiné à générer un flux de gaz comprenant un générateur de flux de gaz (4) et un accélérateur de flux de gaz (5), dans laquelle un premier flux de gaz (2) peut être généré par le générateur de flux de gaz (4) et peut être dirigé vers l'accélérateur de flux de gaz (5) et l'accélérateur de flux de gaz (5) est construit de sorte qu'un second flux de gaz (3) puisse être accéléré par le premier flux de gaz (2), dans laquelle l'accélérateur de flux de gaz (5) est un accélérateur de flux de gaz Coanda utilisant l'effet Coanda pour accélérer le second flux de gaz (3) par le premier flux de gaz (2) du générateur de flux de gaz (4) vers une sortie de gaz (6), dans laquelle le générateur de flux de gaz est un générateur électrohydrodynamique de flux de gaz (4).
